Europäisches Patentamt

European Patent Office

Office européen des brevets

(19)

(11) Veröffentlichungsnummer: **0 195 057**
**B1**

(12) **EUROPÄISCHE PATENTSCHRIFT**

(45) Veröffentlichungstag der Patentschrift: **28.11.90**

(51) Int. Cl.[5]: **A 61 F 6/18**

(21) Anmeldenummer: **85904796.1**

(22) Anmeldetag: **18.09.85**

(86) Internationale Anmeldenummer:
**PCT/DE85/00323**

(87) Internationale Veröffentlichungsnummer:
**WO 86/01709 27.03.86 Gazette 86/07**

(54) **GRÖSSENVARIABLES INTRAUTERINPESSAR UND EMPFÄNGNISVERHÜTENDE VORRICHTUNG.**

(30) Priorität: **18.09.84 DE 3434207**

(43) Veröffentlichungstag der Anmeldung:
**24.09.86 Patentblatt 86/39**

(45) Bekanntmachung des Hinweises auf die
Patenterteilung:
**28.11.90 Patentblatt 90/48**

(84) Benannte Vertragsstaaten:
**AT BE CH DE FR GB IT LI LU NL SE**

(56) Entgegenhaltungen:
**EP-A-0 100 924**
**EP-A-0 117 818**
**DE-A-2 505 104**
**DE-A-2 826 352**
**DE-C- 214 172**
**DE-C- 447 562**
**DE-C- 549 994**
**FR-A- 523 657**
**US-A-1 348 728**
**US-A-3 467 090**
**US-A-3 757 775**
**US-A-4 005 707**

(73) Patentinhaber: **STRUBEL, Bernd-Jochen**
**Strassburger Ring 57**
**D-8700 Würzburg (DE)**
(73) Patentinhaber: **LURZ, Karl-Heinz**
**Seinsheimstrasse 20**
**D-8703 Ochsenfurt (DE)**

(72) Erfinder: **STRUBEL, Bernd-Jochen**
**Strassburger Ring 57**
**D-8700 Würzburg (DE)**
Erfinder: **LURZ, Karl-Heinz**
**Seinsheimstrasse 20**
**D-8703 Ochsenfurt (DE)**

(74) Vertreter: **Münich, Wilhelm, Dr. et al**
**Kanzlei Münich, Steinmann, Schiller**
**Willibaldstrasse 36/38**
**D-8000 München 21 (DE)**

Courier Press, Leamington Spa, England.

## Beschreibung

Die Erfindung bezieht sich auf ein größenvariables Intrauterinpessar, mit zur Anpassung an die Geometrie des Uteruscavums im Uteruscavum verstellbaren Seitenarmen, die an einem zentralen Stabteil angebracht sind, der aus einem Grundkörper und einem Stellzylinder besteht, der ein Innengewinde aufweist, das mit einem Außengewinde auf dem Grundkörper in Eingriff steht, und durch dessen Verschiebung relativ zum Grundteil die Seitenarme verstellbar sind, und mit einem an dem Stabteil angebrachten Kupplungsteil, das während der Anpassung des Intrauterinpessars mit einer getrennten Einstellvorrichtung verbindbar ist, die nach dem Anpaßvorgang wieder entfernbar ist.

Das Design und die Dimension eines Intrauterinpessars müssen in einem bestimmten Verhältnis zum verfügbaren Raum im Uterus stehen. Bei geometrischer Inkompatibilität resultieren zwangsläufig unerwünschte Nebenwirkungen. Geometrische Inkompatibilität ergibt sich entweder durch ein zu kleines Intrauterinpessar oder durch ein zu großes Intrauterinpessar. Die erstgenannte Ursache kann zu einer unerwünschten Schwangerschaft durch Verrutschen des Intrauterinpessars führen. Die an zweiter Stelle genannte Ursache für eine geometrische Inkompatibilität führt zu starkem Drücken, Perforation der Schleimhaut, Schmierblutung oder Schmerzen.

Die Vielzahl der bislang entwickelten Intrauterinpessare zeugt zwar von dem Erkennen dieses Problems, weist aber auch darauf hin, daß dieses Problem bisher nicht gelöst worden ist.

Ein Intrauterinpessar gemäß dem Oberbegriff des Patentanspruchs 1 ist aus der DE-C-214 172 bekannt, das zur Anpassung an die Geometrie des Uteruscavums verstellbare Seitenarme aufweist, die an einem zentralen Stabteil angebracht sind. Die Seitenarme sind in Form eines "Spreizkreuzes" ausgeführt. Zur Verstellung des Spreizkreuzes besteht der zentrale Stabteil aus einem Grundkörper, auf dem ein zylindrisches Teil aufgeschraubt ist, an dessen vorderen Ende das untere Ende des Spreizkreuzes angelenkt ist.

Ferner ist an dem Stabteil ein Kupplungsteil angebracht, mit dem während der Anpassung des Intrauterinpessars eine getrennte Einstellvorrichtung verbindbar ist, die nach dem Anpaßvorgang wieder entfernbar ist.

Dieses bekannte Intrauterinpessar hat aufgrund dieser Ausbildung eine Reihe von Nachteilen:

1. Die das Spreizkreuz bildenden Seitenarme sind "starre Arme", die lediglich miteinander sowie schwenkbar mit dem Grundkörper verbunden sind.

Damit können sich die Seitenarme wie — auch Figur 2 der DE-C 214 172 zeigt, nicht flächig bzw. linienförmig an das Uteruscavum anlegen, sondern liegen lediglich an zwei — bzw. wenn man das vordere Ende des Intrauterinpessars mitrechnet — an drei Stellen "punktförmig" am Uteruscavum auf.

2. Die Seitenarme des aus der DE-C 214 172 bekannten Intrauterinpessars sind beidendig an dem Stabteil angelenkt und stellen damit ein geschlossenes System dar.

3. Das bekannte Intrauterinpessar ist nicht flexibel, so daß es sich - wenn es einmal gespreizt ist - nicht an ein geändertes Uteruscavum anpaßt, so daß die Gefahr des Verrutschens und damit einer ungewollten Schwangerschaft besteht.

4. Das aus der DE-C 214 172 bekannte Intrauterinpessar muß zur Entnahme zurückgestellt werden. Dies bedeutet insbesondere, daß der Arzt zur Entnahme die Einstellvorrichtung wieder an dem Stabteil anbringen muß.

Weiterhin sind durch die US-PS 3 407 806 sowie durch die US-PS 3 410 265 und die US-PS 3 405 711 an die Geometrie des Uteruscavums anpassende Intrauterinpessare bekannt geworden, diese haben jedoch alle den Nachteil, daß durch eine fest eingestellte und nicht veränderbare Federkraft beim Spreizen der Seitenarme bei sehr kleinem Uteruscavum die Gefahr des Drückens, der perforation der Schleimhaut, der Schmierblutung oder Schmerzen allgemein nicht beseitigt werden kann. Bei sehr schwacher Federkraft dagegen besteht die Gefahr bei einem großen Uteruscavum, daß das Intrauterinpessar verrutscht und eine ungewollte Schwangerschaft eintritt.

Auch nach DE-OS 2505104 ist ein Intrauterinpessar bekannt, das einen Stabteil mit flexiblen Seitenarmen aufweist.

Dieses Intrauterinpessar ist jedoch nicht zur Anpassung an die Geometrie des Uteruscavums vorgesehen, sondern es legt sich lediglich durch spezifische Eigenspannung des Kunststoffes je nach Geometrie des Uteruscavums mehr oder weniger stark an dessen Wände an.

Aus der DE-PS 447 562 ist ein Intrauterinpessar mit gegeneinander verschieb-baren Teilen zum Bewegen von Sperrhebeln, jedoch ohne flexible Seitenarme bekannt. Zum Verstellen der Teile sind Sperrzähne vorgesehen, so daß keine Einstellung im eigentlichen Sinne erfolgen kann, sondern nur eine Spreizung.

Aus der DE-PS 362 288 ist zwar ein verstellbarer Pessarkopf bekannt, jedoch in einer äußerst unvorteilhaften Ausgestaltung. Der Pessarkopf ist nicht an die Geometrie des Uteruscavums anpassbar, sondern nur verstellbar, um einigermaßen einfach durch den engen Querschnitt des Eingangs zum Uteruscavum geschoben werden zu können.

Deshalb haben anpaßbare Intrauterinpessare kaum Eingang in die tägliche Routine gefunden.

Der Erfindung liegt deshalb die Aufgabe zugrunde, ein Intrauterinpessar Zur Verfügung zu stellen, welches durch variable Größenänderunugen und optimale Möglichkeiten der Insertionstecknik dem individuellen geometrischen Verhältissen des Uternscavums besser gerecht wird.

Diese Aufgabe wird durch ein Intrauterinpessar gemäß Anspruch 1 gelöst. Weiterbildungen der Erfindung sind in den Unteransprüchen angegeben.

Erfindungsgemäß sind die Seitenarme nur am oberen Rand des Grundkörpers angelenkt und flexibel ausgebildet. Die Arme werden dadurch gespreizt, daß der obere Rand des Stellzylinders gegen diese drückt.

Darüberhinaus ist durch die flexible Ausbildung in Verbindung mit der Anlenkung der Arme nur an einem Ende sichergestellt, daß sich die Arme über ihre ganze Länge an das Uteruscavum anlegen. Hierdurch wird eine große Anlagefläche erreicht, die Unverträglichkeiten etc. weitgehend ausschließt.

Durch dieses größenvariable Intrauterinpessar gemäß der Erfindung ist es nun möglich, nach der Insertion des Intrauterinpessars die Spreizung der Seitenarme optimal an die individuellen Gegebenheiten des Uteruscavums anzupassen.

Es wird dadurch eine größtmögliche Reduzierung der Nebenwirkungen und eine größtmögliche Erhöhung der Sicherheit erzielt.

Die erfindungsgemäße Ausbildung macht es zudem möglich, daß das erfindungsgemäße Intrauterinpessar aus Kunststoff gefertigt werden kann, so daß die Verträglichkeit weiter verbessert wird.

Durch Beibehalten gewohnter Insertionstechniken und durch die äußerst einfache Verstellbarkeit des Intrauterinpessars ist die Handhabung äußerst leicht erlernbar.

Letztlich erlaubt die erfindungsgemäße Ausbildung eine einfache Entnahme des Pessars durch Ziehen beispielsweise an einem an ihm angebrachten Faden, ohne daß ein Rückstellen der Seitenarme erforderlich wäre, da die Seitenarme beim Entnahmevorgang einfach nach oben klappen. Dabei ist es besonders bevorzugt wenn am Grundkörper ein Faden angebracht ist, der eine einfache Entnahme ohne "umständliches Fummeln" gestattet.

Die Erfindung betrifft weiterhin eine empfängnisverhütende Vorrichtung, bestehend aus einem größenvariablen Intrauterinpessar und einer getrennten Einstellvorrichtung.

Im folgenden wird anhand der Zeichnung ein Ausführungsbeispiel der Erfindung beschrieben.

Die Erfindung wird nachstehend anhand eines Ausführungsbeispiels unter Bezugnahme auf die Zeichnung näher beschrieben, in der zeigen:

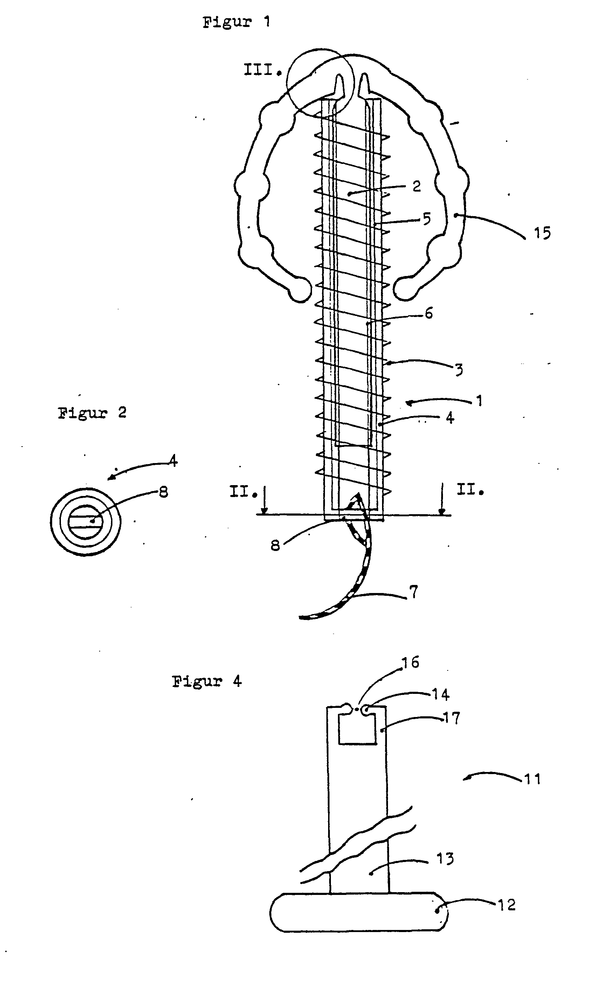

Fig. 1 schematisch in vergrößertem Maßstab ein Intrauterinpessar gemäß der Erfindung

Fig. 2 einen Schnitt entlang der Linie II.-II, in Fig. 1,

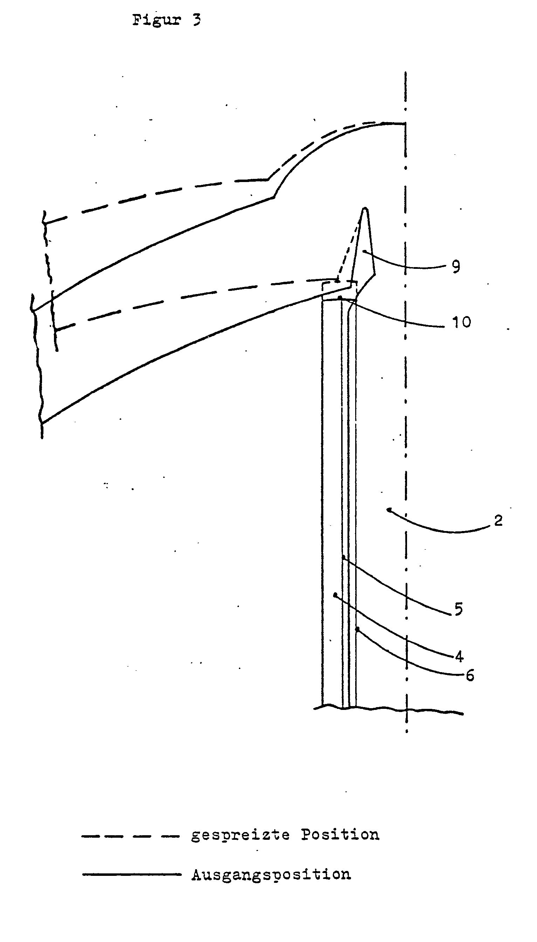

Fig. 3 den vergrößerten Ausschnitt III gemäß Fig. 1 und

Fig. 4 schematisch in vergrößerter Darstellung eine als Drehschlüssel ausgebildete Einstellvorrichtung.

Das Intrauterinpessar besteht aus einem Grundkörper 2, der entlang seines Umfangs ein Außengewinde 6 aufweist.

An diesen Grundkörper 2 sind an seinem oberen Ende zwei ankerförmig ausgebildete Seitenarme 15 angeordnet.

Die Verbindungsstelle des Grundkörpers mit den Seitenarmen weist eine Engstelle auf, die durch eine Kerbe 9 erzeugt wird.

Auf dem Grundkörper befindet sich koaxial ein Stellzylinder 4, der ein Innengewinde 5 aufweist, das mit dem Außengewinde 6 des Grundkörpers 2 in Eingriff steht. Der Stellzylinder 4 weist an seinem unteren Ende einen als Kupplungsteil wirkenden Steg 8 auf. An diesem Steg 8 kann zudem der Faden 7 befestigt werden. Auf dem Stellzylinder 4 ist eine Kupferspirale 3 befestigt.

Die Figur 4 zeigt eine als Drehschlüssel ausgebildete Einstellvorrichtung 11. Die Einstellvorrichtung 11 besteht aus einem Griff 12 und einem Schaft 13. An dem Griff 12 entfernten Ende des Schaftes 13 sind zwei Zapfen 17 angeordnet, die zwei nach innen weisende Schnappköpfe 14 aufweisen. Zwischen den Schnappköpfen 14 ist ein an den Durchmesser des Kupplungsteiles 8 angepaßter Abstand 16 vorhanden.

Im folgenden wird kurz die Wirkungsweise des größenvariablen Intrauterinpessars beschrieben.

Bei der Insertion des größenvariablen Intrauterinpessars ist die Einstellvorrichtung 11 über das Kupplungsteil 8 mit dem Intrauterinpessar kardanisch verbunden. Nachdem das Intrauterinpessar wie gewohnt in das Uteruscavum insertiert wurde, wird durch Drehen der Einstellvorrichtung 11 der obere Rand 10 des Stellzylinders 4 gegen die Seitenarme 15 des Intrauterinpessar gedrückt. Durch diesen Druck bewegen sich die Seitenarme in Richtung der Uteruswand bis zur Anlage an die Uteruswand.

Durch Bewegen des Intrauterinpessars während der Anpassung kann der ausführende Arzt den Zeitpunkt des optimalen Sitzes des Intrauterinpessars feststellen.

Nachdem das Intrauterinpessar optimal im Uteruscavum festgelegt wurde, wird durch leichten Zug die Einstellvorrichtung 11 vom Kupplungsteil 8 getrennt und entfernt.

Durch dieses größenvariable Intrauterinpessar ist es erstmals möglich, mit einer einzigen Größe alle geometrischen Verhältnisse des Uteruscavums optimal abdecken zu können und somit die Nebenwirkungen bestmöglich zu reduzieren und die Sicherheit zu erhöhen.

**Patentansprüche**

1. Größenvariables Intrauterinpessar, mit zur Anpassung an die Geometrie des Uteruscavums im Uteruscavum verstellbaren Seitenarmen (15), die an einem zentralen Stabteil angebracht sind, der aus einem Grundkörper (2) und einem Stellzylinder (4) besteht, der ein Innengewinde aufweist, das mit einem Außengewinde auf dem Grundkörper in Eingriff steht, und durch dessen Verschiebung relativ zum Grundteil die Seitenarme verstellbar sind, und

mit einem an dem Stabteil (1) angebrachten Kupplungsteil (8), das während der Anpassung des Intrauterinpessars mit einer getrennten Einstellvorrichtung (11) verbindbar ist, die nach dem Anpaßvorgang wieder entfernbar ist,

dadurch gekennzeichnet, daß die Seitenarme (15) nur am oberen Rand des Grundkörpers (2) angelenkt und flexibel ausgebildet sind,

daß zum beliebig starken Spreizen der Arme (15) zur Anpassung an die Geometrie des Uteruscavums der obere Rand des Stellzylinders (4) gegen die Arme drückt, daß sowohl der Stabteil (1) als auch die Seitenarme (15) aus Kunststoff bestehen, und

daß der Grundkörper (2) eine Befestigungsmöglichkeit für einen Faden aufweist.

2. Intrauteinpessar nach Anspruch 1, dadurch gekennzeichnet, daß der zentrale Stabteil (1) mit einer insbesondere aus Kupfer bestehenden Spirale umwickelt ist.

3. Intrauterinpessar nach Anspruch 1 oder 2, dadurch gekennzeichnet, daß an der Verbindungsstelle des Grundkörpers (2) mit den Seitenarmen (15) eine Engstelle (9) vorgesehen ist.

4. Intrauterinpessar nach einem der Ansprüche 1 bis 3, dadurch gekennzeichnet, daß das Kupplungsteil (8) als Steg ausgebildet ist.

## Revendications

1. Dispositif intra-utérin à grandeur variable, comprenant des branches latérales (15) adaptables à la géometrie de la cavité utérine au dedans de la cavité utérine, lesdites branches étant fixées à un élément-baguette central composé d'un corps de base (2) et d'un cylindre d'ajustement (4) pourvu d'un taraudage en prise avec un filet extérieur sur le corps de base et déplaçable relativement au corps de base pour ajuster les branches latérales, et comprenant en plus un élément coupleur (8) attaché à l'élément-baguette et approprié pour le raccord à un dispositif d'ajustage séparé (11) au cours de l'opération d'adaptation du dispositif intra-utérin, ledit dispositif d'ajustage étant enlevable après l'opération d'adaptation, caractérisé en ce que lesdites branches latérales (15) ne sont articulées qu'au bord supérieur du corps de base (2) et sont flexibles, que ledit bord supérieur dudit cylindre d'ajustage (4) pousse contre lesdites branches (15) pour leur écartement à volonté afin de les adapter à la géométrie de la cavité utérine, que ledit élément-baguette (1) ainsi que lesdites branches latérales (15) sont fabriqués en matière plastique, et que ledit corps de base (2) présente des moyens d'attache pour un fil.

2. Dispositif intra-utérin selon la Revendication 1, caractérisé en ce que ledit élément-baguette central (1) est enveloppé par une spirale en cuivre.

3. Dispositif intra-utérin selon la Revendication 1 ou 2, caractérisé en ce qu'à la jonction entre ledit corps de base (2) et les branches latérales (15) un étranglement (9) est pourvu.

4. Dispositif intra-utérin selon quelconque des Revendications 1 à 3, caractérisé en ce que ledit élément accoupleur (8) est configuré sous forme d'une traverse.

## Claims

1. Intra-uterine pessary variable in size, comprising lateral arms (15) adjustable in the uterine cavity for adaptation to the geometry of the uterine cavity, which arms are attached at a central rod element consisting of a basic body (2) and an adjusting cylinder (4) including an internal thread in engagement with an external thread on said basic body, said arms being adjustable by displacement of said cylinder relative to the basic body and furthermore comprising a coupler element (8) attached at said rod element (1), which during adaptation of the intra-uterine pessary may be coupled to a separate adjusting device (11) that may be removed again after the adaptation procedure,

characterized in that said lateral arms (15) are articulated only at the upper edge of said basic body (2) and present a flexible design, that the upper edge of said adjusting cylinder (4) presses against said arms so as to achieve an optional extent of spreading of said arms (15) for matching to the geometry of the uterus cavity, that both said rod element (1) and said lateral arms (15) consist of plastic material, and that said basic body (2) comprises provisions for attachment of a thread.

2. Intra-uterine pessary according to Claim 1, characterized in that said central rod element (1) is wire-wrapped by a spiral consisting of copper in particular.

3. Intra-uterine pessary according to Claim 1 or 2, characterized in that at the junction between said basic body (2) and said lateral arms (15) a bottle-neck area (9) is provided.

4. Intra-uterine pessary according to any of Claims 1 to 3, characterized in that said coupler element (8) presents a crossbar design.

Figur 1

III.

2

5

15

6

3

1

4

Figur 2

4

8

II.

II.

8

7

Figur 4

16

14

17

11

13

12

Figur 3

9

10

2

5

4

6

— — — — — gespreizte Position

————————— Ausgangsposition